# EUROPEAN PATENT APPLICATION

(11) **EP 1 847 537 A1**
(43) Date of publication of application: **24.10.2007**
(21) Application number: 06112897.1
(22) Date of filing: 21.04.2006
(51) Int. Cl.: C07D 401/12, A61K 31/444, A61P 7/02

(54) **Dicarboxamide derivatives**

(71) Applicant: F. HOFFMANN-LA ROCHE AG, 4070 Basel (CH)
(72) Inventor: The designation of the inventor has not yet been filed
(74) Representative: Niizuma, Yo

(57) **Abstract**

The invention is concerned with dicarboxamide derivatives of formula (I) 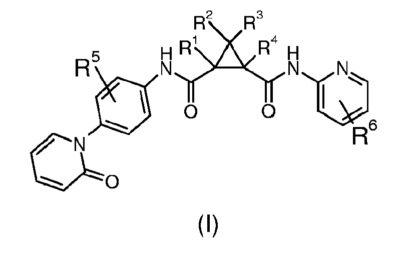 wherein R¹, R², R³, R⁴, R⁵ and R⁶ are as defined in the specification, as well as physiologically acceptable salts thereof. These compounds inhibit the coagulation factor Xa and can be used as medicaments.

## Description

The invention is concerned with dicarboxamide derivatives of formula (I) 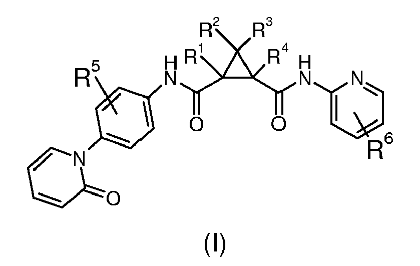 wherein
R² and R³ are independently from each other hydrogen, C₁₋₆ alkyl, carboxyl, C₁₋₆ alkoxycarbonyl, carbamoyl, mono- or di-substituted amino-carbonyl, optionally substituted aryl carbonyl, optionally substituted heterocyclylcarbonyl, optionally substituted heteroarylcarbonyl, optionally substituted aryl, optionally substituted heteroaryl, optionally substituted heterocyclyl, hydroxyl C₁₋₆ alkyl, halo C₁₋₆ alkyl, cyano C₁₋₆ alkyl, C₁₋₆ alkoxy C₁₋₆ alkyl, amino C₁₋₆ alkyl, mono- or di-substituted amino-C₁₋₆ alkyl, optionally substituted aryl C₁₋₆ alkyl, optionally substituted heterocyclyl C₁₋₆ alkyl, optionally substituted heteroaryl C₁₋₆ alkyl, optionally substituted aryl C₁₋₆ alkoxy C₁₋₆ alkyl, optionally substituted heteroaryl C₁₋₆ alkoxy C₁₋₆ alkyl, optionally substituted heterocyclyl C₁₋₆ alkoxy C₁₋₆ alkyl,
R¹ and R⁴ are, independently from each other hydrogen, C₁₋₆ alkyl, cyano, C₁₋₆ alkoxycarbonyl, C₂₋₆ alkenyloxycarbonyl, C₂₋₆ alkynyloxycarbonyl, hydroxyl C₁₋₆ alkyl, C₁₋₆ alkoxycarbonyl, carboxyl, mono- or di-C₁₋₆ alkyl substituted amino-carbonyl, aminocarbonyl, optionally substituted heterocyclyl carbonyl, optionally substituted heteroaryl carbonyl or optionally substituted aryl carbonyl,
R⁵ and R⁶ are, independently from each other, are chlorine, fluorine or bromine;
or pharmaceutically acceptable salts thereof.
R² and R³ are preferably, independently from each other, hydrogen, C₁₋₆ alkyl, carboxyl, C₁₋₆ alkoxycarbonyl, carbamoyl, mono- or di-substituted amino-carbonyl, optionally substituted heterocyclylcarbonyl, optionally substituted heteroarylcarbonyl, aryl, optionally substituted heteroaryl, optionally substituted heterocyclyl, hydroxyl C₁₋₆ alkyl, C₁₋₆ alkoxy C₁₋₆ alkyl, amino C₁₋₆ alkyl, mono- or di-substituted amino-C₁₋₆ alkyl, optionally substituted heterocyclyl C₁₋₆ alkyl or optionally substituted heteroaryl C₁₋₆ alkyl,
R¹ and R⁴ are preferably, independently from each other, hydrogen or C₁₋₆ alkyl.

Further, the invention is concerned with a process for the manufacture of such compounds, pharmaceutical preparations which contain such compounds as well as the use of these compounds for the production of pharmaceutical preparations.

The compounds of the present invention are active compounds and inhibit the coagulation factor Xa. These compounds consequently influence blood coagulation. They therefore inhibit the formation of thrombin and can be used for the treatment and/or prevention of thrombotic disorders, such as amongst others, arterial and venous thrombosis, deep vein thrombosis, peripheral arterial occlusive disease (PAOD), unstable angina pectoris, myocardial infarction, coronary artery disease, pulmonary embolism, stroke (cerebral thrombosis) due to atrial fibrillation, inflammation and arteriosclerosis. They have potentially benefit in the treatment of acute vessel closure associated with thrombolytic therapy and restenosis, e.g. after transluminal coronary angioplasty (PTCA) or bypass grafting of the coronary or peripheral arteries and in the maintenance of vascular access patency in long term hemodialysis patients. F.Xa inhibitors of this invention may form part of a combination therapy with an anticoagulant with a different mode of action or with a platelet aggregation inhibitor or with a thrombolytic agent. Furthermore, these compounds have an effect on tumour cells and prevent metastases. They can therefore also be used as antitumour agents.

Other inhibitors of factor Xa, which are not structurally related to the compounds of the present invention, had previously been suggested for the inhibition of the formation of thrombi and for the treatment of related diseases (WO 03/045912). However, there is still a need for novel factor Xa inhibitors which exhibit improved pharmacological properties, e.g. an improved selectivity towards coagulation factor Xa.

The present invention provides the novel compounds which are factor Xa inhibitors. The compounds of the present invention unexpectedly inhibit coagulation factor Xa and also exhibit improved pharmacological properties compared to other compounds already known in the art.

Compounds of the present invention can form pharmaceutically acceptable acid addition salts. Examples of such pharmaceutically acceptable salts are salts of the compounds with physiologically compatible mineral acids, such as hydrochloric acid, sulphuric acid, sulphurous acid or phosphoric acid; or with organic acids, such as methanesulphonic acid, p-toluenesulphonic acid, acetic acid, lactic acid, trifluoroacetic acid, citric acid, fumaric acid, maleic acid, tartaric acid, succinic acid or salicylic acid. The term "pharmaceutically acceptable salts" refers to such salts. Acid addition salts as described above are preferred.

Unless otherwise indicated, the following definitions are set forth to illustrate and define the meaning and scope of the various terms used to describe the invention herein.

The term "C₁₋₆ alkyl" means a branched or straight-chain monovalent alkyl radical, having one to six carbon atoms. This term is further exemplified by such radicals as methyl, ethyl, n-propyl, isopropyl, n-butyl, s-butyl, t-butyl. Methyl is more preferred.

The term "C₂₋₆ alkynyl", alone or in combination with other groups, means a straight-chain or branched hydrocarbon residue comprising a tripple bond and 2 to 6 carbon atoms, such as e.g. 2-propinyl.

The term"halo C₁₋₆ alkyl" means C₁₋₆ alkyl substituted by one or more same or different halogen atoms independently selected from the group consisting of chlorine, fluorine and bromine.

The term "cyano C₁₋₆ alkyl" means C₁₋₆ alkyl substituted by one or more cyano groups, preferably one cyano group.

The term "hydroxy C₁₋₆ alkyl" means C₁₋₆ alkyl substituted by one or more hydroxy groups, preferably one or two hydroxy groups.

The term "C₃₋₇ cycloalkyl", alone or in combination with other groups, means a saturated monovalent cyclic hydrocarbon radical of three to seven ring carbons, e.g., cyclopropyl, cyclobutyl, cyclohexyl.

The term "C₁₋₆ alkoxy", alone or in combination with other groups, means the group R'-O-, wherein R' is a C₁₋₆ alkyl.

The term "C₂₋₆ alkenyl", alone or in combination with other groups, means a straight-chain or branched hydrocarbon residue comprising an olefinic bond, having two to six carbon atoms, such as e.g. ethenyl, 2-propenyl.

The term "aryl", alone or in combination with other groups, means a phenyl or a naphthyl group, preferably a phenyl group. The term "optionally substituted aryl" means an aryl group described above, which is optionally substituted by one to five , preferably one to three substituents independently selected from the group consisting of halogen, hydroxy, C₁₋₆ alkyl, halo C₁₋₆ alkyl, C₁₋₆ alkoxy, C₁₋₆ alkyl sulfonyl, C₁₋₆ alkyl sulfinyl, C₁₋₆ alkylthio, amino, amino C₁₋₆ alkyl, mono- or di-substituted amino-C₁₋₆ alkyl, nitro, cyano, acyl, carbamoyl, mono- or di-substituted amino, aminocarbonyl, mono- or di-substituted amino-carbonyl, aminocarbonyl C₁₋₆ alkoxy, mono- or di-substituted amino-carbonyl-C₁₋₆ alkoxy, hydroxy- C₁₋₆ alkyl, carboxyl, C₁₋₆ alkoxy carbonyl, aryl C₁₋₆ alkoxy, heteroaryl C₁₋₆ alkoxy, heterocyclyl C₁₋₆ alkoxy, C₁₋₆ alkoxycarbonyl C₁₋₆ alkoxy, carbamoyl C₁₋₆ alkoxy and carboxyl C₁₋₆ alkoxy, preferably selected from the group consisting of halogen, hydroxy, C₁₋₆ alkyl, halo C₁₋₆ alkyl, C₁₋₆ alkoxy, C₁₋₆ alkyl sulfonyl, C₁₋₆ alkyl sulfinyl, C₁₋₆ alkylthio, amino, mono-C₁₋₆ alkyl substituted amino, di-C₁₋₆ alkyl substituted amino, amino C₁₋₆ alkyl, mono-C₁₋₆ alkyl substituted amino-C₁₋₆ alkyl, di-C₁₋₆ alkyl substituted amino-C₁₋₆ alkyl, nitro and cyano.

The term "heterocyclyl", alone or combination with other groups, means nonaromatic monocyclic radicals of three to eight ring atoms in which one or two ring atoms are heteroatoms selected from N, O, or S(O)ₙ (where n is an integer from 0 to 2), the remaining ring atoms being C. One or two ring carbon atoms of heterocyclyl group may be replaced with a carbonyl group. The term "optionally substituted heterocyclyl" means a heterocyclyl group described above, which is optionally substituted independently by one, two, or three substituents, preferably one or two substituents selected from the group consisting of halogen, hydroxy, C₁₋₆ alkyl, halo C₁₋₆ alkyl, C₁₋₆ alkoxy, C₁₋₆ alkyl sulfonyl, C₁₋₆ alkyl sulfinyl, C₁₋₆ alkylthio, amino, amino C₁₋₆ alkyl, mono- or di-substituted amino-C₁₋₆ alkyl, nitro, cyano, acyl, carbamoyl, mono- or di-substituted amino, aminocarbonyl, mono- or di-substituted amino-carbonyl, aminocarbonyl C₁₋₆ alkoxy, mono- or di-substituted amino-carbonyl-C₁₋₆ alkoxy, hydroxy- C₁₋₆ alkyl, carboxyl, C₁₋₆ alkoxy carbonyl, aryl C₁₋₆ alkoxy, heteroaryl C₁₋₆ alkoxy, heterocyclyl C₁₋₆ alkoxy, C₁₋₆ alkoxycarbonyl C₁₋₆ alkoxy, carbamoyl C₁₋₆ alkoxy and carboxyl C₁₋₆ alkoxy, preferably selected from the group consisting of halogen, hydroxy, C₁₋₆ alkyl, halo C₁₋₆ alkyl, C₁₋₆ alkoxy, acyl, C₁₋₆ alkyl sulfonyl, C₁₋₆ alkyl sulfinyl, C₁₋₆ alkylthio, amino, mono-C₁₋₆ alkyl substituted amino, di-C₁₋₆ alkyl substituted amino, amino C₁₋₆ alkyl, mono-C₁₋₆ alkyl substituted amino-C₁₋₆ alkyl, di-C₁₋₆ alkyl substituted amino-C₁₋₆ alkyl, nitro, carbamoyl, mono- or di-substituted amino-carbonyl, hydroxy- C₁₋₆ alkyl, carboxyl, C₁₋₆ alkoxy carbonyl and cyano, more preferably selected from the group consisting of halogen, hydroxy, C₁₋₆ alkyl, halo C₁₋₆ alkyl, C₁₋₆ alkoxy, C₁₋₆ alkyl sulfonyl, C₁₋₆ alkyl sulfinyl, C₁₋₆ alkylthio, amino, mono-C₁₋₆ alkyl substituted amino, di-C₁₋₆ alkyl substituted amino, amino C₁₋₆ alkyl, mono-C₁₋₆ alkyl substituted amino-C₁₋₆ alkyl, di-C₁₋₆ alkyl substituted amino-C₁₋₆ alkyl, nitro and cyano.

The term "heteroaryl", alone or combination with other groups, means a monocyclic or bicyclic radical of 5 to 12 ring atoms having at least one aromatic ring containing one, two, or three ring heteroatoms selected from N, O, and S, the remaining ring atoms being C, with the understanding that the attachment point of the heteroaryl radical will be on an aromatic ring. One or two ring carbon atoms of heteroaryl group may be replaced with a carbonyl group. The term "optionally substituted heteroaryl" means a heteroaryl group described above, which is optionally substituted independently with one, two, or three substituents, preferably one or two substituents selected from the group consisting of halogen, hydroxy, C₁₋₆ alkyl, halo C₁₋₆ alkyl, C₁₋₆ alkoxy, C₁₋₆ alkyl sulfonyl, C₁₋₆ alkyl sulfinyl, C₁₋₆ alkylthio, amino, amino C₁₋₆ alkyl, mono- or di-substituted amino-C₁₋₆ alkyl, nitro, cyano, acyl, carbamoyl, mono- or di-substituted amino, aminocarbonyl, mono- or di-substituted amino-carbonyl, aminocarbonyl C₁₋₆ alkoxy, mono- or di-substituted amino-carbonyl-C₁₋₆ alkoxy, hydroxy- C₁₋₆ alkyl, carboxyl, C₁₋₆ alkoxy carbonyl, aryl C₁₋₆ alkoxy, heteroaryl C₁₋₆ alkoxy, heterocyclyl C₁₋₆ alkoxy, C₁₋₆ alkoxycarbonyl C₁₋₆ alkoxy, carbamoyl C₁₋₆ alkoxy and carboxyl C₁₋₆ alkoxy, preferably selected from the group consisting of halogen, hydroxy, C₁₋₆ alkyl, halo C₁₋₆ alkyl, C₁₋₆ alkoxy, C₁₋₆ alkyl sulfonyl, C₁₋₆ alkyl sulfinyl, C₁₋₆ alkylthio, amino, mono-C₁₋₆ alkyl substituted amino, di-C₁₋₆ alkyl substituted amino, amino C₁₋₆ alkyl, mono-C₁₋₆ alkyl substituted amino-C₁₋₆ alkyl, di-C₁₋₆ alkyl substituted amino-C₁₋₆ alkyl, nitro, carbamoyl, mono- or di-substituted amino-carbonyl, hydroxy- C₁₋₆ alkyl, carboxyl, C₁₋₆ alkoxy carbonyl and cyano.

The term "optionally substituted phenyl" means a phenyl group optionally substituted by one to five substituents, preferably one to three substituents independently selected from the group consisting of halogen, hydroxy, C₁₋₆ alkyl, halo C₁₋₆ alkyl, C₁₋₆ alkoxy, C₁₋₆ alkyl sulfonyl, C₁₋₆ alkyl sulfinyl, C₁₋₆ alkylthio, amino, amino C₁₋₆ alkyl, mono- or di-substituted amino-C₁₋₆ alkyl, nitro, cyano, acyl, carbamoyl, mono- or di-substituted amino, aminocarbonyl, mono- or di-substituted amino-carbonyl, aminocarbonyl C₁₋₆ alkoxy, mono- or di-substituted amino-carbonyl-C₁₋₆ alkoxy, hydroxy- C₁₋₆ alkyl, carboxyl, C₁₋₆ alkoxy carbonyl, aryl C₁₋₆ alkoxy, heteroaryl C₁₋₆ alkoxy, heterocyclyl C₁₋₆ alkoxy, C₁₋₆ alkoxycarbonyl C₁₋₆ alkoxy, carbamoyl C₁₋₆ alkoxy and carboxyl C₁₋₆ alkoxy, preferably selected from the group consisting of halogen, hydroxy, C₁₋₆ alkyl, halo C₁₋₆ alkyl, C₁₋₆ alkoxy, C₁₋₆ alkyl sulfonyl, C₁₋₆ alkyl sulfinyl, C₁₋₆ alkylthio, amino, mono-C₁₋₆ alkyl substituted amino, di-C₁₋₆ alkyl substituted amino, amino C₁₋₆ alkyl, mono-C₁₋₆ alkyl substituted amino-C₁₋₆ alkyl, di-C₁₋₆ alkyl substituted amino-C₁₋₆ alkyl, nitro and cyano.

The term "mono-substituted amino" and "di-substituted amino", alone or combination with other groups, mean -NHR and -NRR' respectively, in which R and R' are independently selected from the group consisting of hydroxy, C₁₋₆ alkyl, hydroxy C₁₋₆ alkyl, C₁₋₆ alkoxy C₁₋₆ alkyl, carbamoyl C₁₋₆ alkyl, halo C₁₋₆ alkyl, C₃₋₇ cycloalkyl, C₃₋₇ cycloalkyl C₁₋₆ alkyl, C₁₋₆ alkoxy, C₁₋₆ alkyl sulfonyl, C₁₋₆ alkyl sulfinyl, C₁₋₆ alkylthio, mono- or di-C₁₋₆ alkyl substituted amino-sulfonyl, mono- or di-C₁₋₆ alkyl substituted amino-sulfinyl, mono- or di-C₁₋₆ alkyl substituted amino-thio, mono- or di-C₁₋₆ alkyl substituted amino-C₁₋₆ alkyl, mono- or di-C₁₋₆ alkyl substituted aminocarbonyl-C₁₋₆ alkyl, acyl, halo C₁₋₆ alkylcarbonyl and C₁₋₆ alkoxycarbonyl, preferably selected from the group consisting of hydroxy, C₁₋₆ alkyl, hydroxy C₁₋₆ alkyl, C₁₋₆ alkoxy C₁₋₆ alkyl, carbamoyl C₁₋₆ alkyl, halo C₁₋₆ alkyl, C₃₋₇ cycloalkyl, C₃₋₇ cycloalkyl C₁₋₆ alkyl, C₁₋₆ alkoxy, C₁₋₆ alkyl sulfonyl, C₁₋₆ alkyl sulfinyl, C₁₋₆ alkylthio, mono- or di-C₁₋₆ alkyl substituted amino-sulfonyl, mono- or di-C₁₋₆ alkyl substituted amino-sulfinyl, mono- or di-C₁₋₆ alkyl substituted amino-thio, mono- or di-C₁₋₆ alkyl substituted amino-C₁₋₆ alkyl, mono- or di-C₁₋₆ alkyl substituted aminocarbonyl-C₁₋₆ alkyl, acyl and C₁₋₆ alkoxycarbonyl, preferably selected from the group consisting of hydroxy, C₁₋₆ alkyl, hydroxy C₁₋₆ alkyl, halo C₁₋₆ alkyl, C₃₋₇ cycloalkyl, C₃₋₇ cycloalkyl C₁₋₆ alkyl, C₁₋₆ alkoxy, C₁₋₆ alkyl sulfonyl, C₁₋₆ alkyl sulfinyl, C₁₋₆ alkylthio, mono- or di-C₁₋₆ alkyl substituted amino-sulfonyl, mono- or di-C₁₋₆ alkyl substituted amino-sulfinyl, mono- or di-C₁₋₆ alkyl substituted amino-thio, acyl and C₁₋₆ alkoxycarbonyl.

The term "acyl", alone or combination with other groups, means -C(=O) R, in which R is H or C₁₋₆ alkyl.

Preferred radicals for the chemical groups whose definitions are given above are those specifically exemplified in Examples.

"Pharmaceutically acceptable excipient" means an excipient that is useful in preparing a pharmaceutical composition that is generally safe, non-toxic and neither biologically nor otherwise undesirable, and includes excipient that is acceptable for veterinary use as well as human pharmaceutical use. A "pharmaceutically acceptable excipient" as used in the specification and claims includes both one and more than one such excipient.

Compounds that have the same molecular Formula but differ in the nature or sequence of bonding of their atoms or the arrangement of their atoms in space are termed "isomers." Isomers that differ in the arrangement of their atoms in space are termed "stereoisomers". Stereoisomers that are not mirror images of one another are termed "diastereomers" and those that are non-superimposable mirror images of each other are termed "enantiomers". When a compound has an asymmetric center, for example, if a carbon atom is bonded to four different groups, a pair of enantiomers is possible. An enantiomer can be characterized by the absolute configuration of its asymmetric center and is described by the R- and S-sequencing rules of Cahn, Ingold and Prelog, or by the manner in which the molecule rotates the plane of polarized light and designated as dextrorotatory or levorotatory (i.e., as (+) or (-)-isomers respectively). A chiral compound can exist as either individual enantiomer or as a mixture thereof. A mixture containing equal proportions of the enantiomers is called a "racemic mixture".

As described above, the compounds of the present invention are active compounds and inhibit the coagulation factor Xa. These compounds consequently influence both platelet activation which is induced by this factors and plasmatic blood coagulation. They therefore inhibit the formation of thrombin and can be used for the treatment and/or prevention of thrombotic disorders, such as, amongst others, arterial and venous thrombosis, deep vein thrombosis, peripheral arterial occlusive disease (PAOD), unstable angina pectoris, myocardial infarction, coronary artery disease, pulmonary embolism, stroke (cerebral thrombosis) due to atrial fibrillation, inflammation and arteriosclerosis. The compounds of the present invention can also be used in the treatment of acute vessel closure associated with thrombolytic therapy and restenosis, e.g. after transluminal coronary angioplasty (PTCA) or bypass grafting of the coronary or peripheral arteries and in the maintenance of vascular access patency in long term hemodialysis patients. F.Xa inhibitors of this invention may form part of a combination therapy with an anticoagulant with a different mode of action or with a platelet aggregation inhibitor or with a thrombolytic agent. Furthermore, these compounds have an effect on tumour cells and prevent metastases. They can therefore also be used as antitumour agents.

Prevention and/or treatment of thrombotic disorders, particularly arterial or deep vein thrombosis, is the preferred indication.

The invention therefore also relates to pharmaceutical compositions comprising a compound of the present invention and a pharmaceutically acceptable excipient.

The invention likewise embraces compounds of the present invention for use as therapeutically active substances, especially as therapeutically active substances for the treatment and/or prophylaxis of diseases which are associated with the coagulation factor Xa, particularly as therapeutically active substances for the treatment and/or prophylaxis of thrombotic disorders, arterial thrombosis, venous thrombosis, deep vein thrombosis, peripheral arterial occlusive disease, unstable angina pectoris, myocardial infarction, coronary artery disease, pulmonary embolism, stroke due to atrial fibrillation, inflammation, arteriosclerosis, acute vessel closure associated with thrombolytic therapy or restenosis, and/or tumour

In another preferred embodiment, the invention relates to a method for the therapeutic and/or prophylactic treatment of diseases which are associated with the coagulation factor Xa, particularly for the therapeutic and/or prophylactic treatment of thrombotic disorders, arterial thrombosis, venous thrombosis, deep vein thrombosis, peripheral arterial occlusive disease, unstable angina pectoris, myocardial infarction, coronary artery disease, pulmonary embolism, stroke due to atrial fibrillation, inflammation, arteriosclerosis, acute vessel closure associated with thrombolytic therapy or restenosis, and/or tumour, which method comprises administering a compound as defined above to a human being or animal.

The invention also embraces the use of compounds as defined above for the therapeutic and/or prophylactic treatment of diseases which are associated with the coagulation factor Xa, particularly for the therapeutic and/or prophylactic treatment of thrombotic disorders, arterial thrombosis, venous thrombosis, deep vein thrombosis, peripheral arterial occlusive disease, unstable angina pectoris, myocardial infarction, coronary artery disease, pulmonary embolism, stroke due to atrial fibrillation, inflammation, arteriosclerosis, acute vessel closure associated with thrombolytic therapy or restenosis, and/or tumour.

The invention also relates to the use of compounds of the present invention for the preparation of medicaments for the therapeutic and/or prophylactic treatment of diseases which are asscociated with the coagulation factor Xa, particularly for the therapeutic and/or prophylactic treatment of thrombotic disorders, arterial thrombosis, venous thrombosis, deep vein thrombosis, peripheral arterial occlusive disease, unstable angina pectoris, myocardial infarction, coronary artery disease, pulmonary embolism, stroke due to atrial fibrillation, inflammation, arteriosclerosis, acute vessel closure associated with thrombolytic therapy or restenosis, and/or tumour. Such medicaments comprise a compound of the present invention.

The inhibition of the coagulation factor Xa by the compounds of the present invention can be demonstrated with the aid of a chromogenic peptide substrate assay as described hereinafter.

Factor Xa activity was measured spectrophotometrically in microtiter plates in a final volume of 150 µl using the following conditions: Inhibition of human factor Xa (Enzyme Research Laboratories) was tested at an enzyme concentration of 3 nM using the chromogenic substrate S-2222 (Chromogenix AB, Mölndal, Sweden) at 200 nM. The reaction kinetics of the enzyme and the substrate were linear with both time and the enzyme concentration. The inhibitors were dissolved in DMSO and tested at various concentrations up to 100 µM. The inhibitors were diluted using HNPT buffer consisting of HEPES 100mM, NaCl 140mM, PEG 6000 0.1% and Tween 80 0.02%, pH 7.8. The cleavage of S-2222 by human factor Xa was followed at 405 nm for 5 minutes at room temperature. The velocity of the reaction was determined by the autoreader from the slope of the linear regression fit to 7 time points (1 minute). The initial velocity for each inhibitor concentration was determined by the slope of at least 4 time points in the linear phase by a linear regression fit (mOD/min²). Apparent dissociation constants Kᵢ were calculated according to Cheng and Prusoff [Cheng, Y. C.; Prusoff, W. H. Relationship between the inhibition constant (Ki) and the concentration of the inhibitor that causes 50 percent inhibition (IC50) of an enzyme reaction. Biochem. Pharmacol. 1973, 22, 3099-3108.] based on the IC₅₀ and the respective Kₘ, determined previously (Kᵢ= IC₅₀/ (1+S/Kₘ)). The Kₘ for the substrate used was determined under the conditions of the test with at least 5 substrate concentrations ranging from 0.5 to 15 times Kₘ. [Lottenberg R, Hall JA, Blinder M, Binder EP, Jackson CM., The action of thrombin on peptide p-nitroanilide substrates. Substrate selectivity and examination of hydrolysis under different reaction conditions. Biochim Biophys Acta. 1983 Feb 15; 742(3):539-57]. according to Eadie [Eadie G.S. The inhibition of cholinesterase by physostigmine and prostigmine. J. Biol. Chem. 1942, 146, 85-93.]. The Kₘ for S-2222 amounted to 613 µM.

The activity of the low molecular weight substances can, moreover, be characterized in the "prothrombin time" (PT) clotting test. The substances are prepared as a 10 mM solution in DMSO and thereafter made up to the desired dilution in the same solvent. Thereafter, 0.25 ml of human plasma (obtained from whole blood anticoagulated with 1/10 volume of 108 mM Na citrate) was placed in the instrument-specific sample container. In each case 5 µl of each dilution of the substance-dilution series was then mixed with the plasma provided. This plasma/inhibitor mixture was incubated at 37°C for 2 minutes. Thereafter, there were pipetted to the semi-automatic device (ACL, Automated Coagulation Laboratory (Instrument Laboratory)) 50 µl of plasma/ inhibitor mixture in the measurement container. The clotting reaction was initiated by the addition of 0.1 ml of Dade®Innovin®(recombinant human tissue factor combined with calcium buffer and synthetic phospholipids, Dade Behring, Inc., Cat. 84212-50). The time up to the fibrin cross-linking was determined photooptically from the ACL. The inhibitor concentration, which brought about a doubling of the PT clotting time, was determined by fitting the data to an exponential regression (XLfit).

The compounds of the present invention can furthermore be characterised by the Activated Partial Thromboplastin time (aPTT). This coagulation test can e.g. be run on the ACL 300 Coagulation System (Instrumentation Laboratory) automatic analyzer. The substances are prepared as a 10 mM solution in DMSO and thereafter made up to the desired dilution in the same solvent. The test is performed with the Dade®Actin®FS Activated PTT reagent (purified soy phosphatides in 1.0x10⁻⁴M ellagic acid, stabilizers and preservative, Dade Behring, Inc., Cat. B4218-100) Thereafter, 0.25 ml aliquots of human plasma (obtained from whole blood anticoagulated with 1/10 volume of 108 mM Na citrate) are spiked with 5 µl of test compound in at least 6 concentrations. 50 µl plasma at 4°C containing 1/50 vol. inhibitor in solvent are incubated with 50 µl Dade®Actin®FS Activated PTT reagent in water at 37°C for 3 min., then 50 µl CaC12.2H2O 25 mM in water at 37°C are added. The time up to the fibrin cross-linking was determined photooptically from the ACL. The inhibitor concentration, which brought about a doubling of the APTT clotting time, was determined by fitting the data to an exponential regression (XLfit).

The Ki values of the active compounds of the present invention preferably amount to about 0.001 to 50 µM, especially about 0.001 to 1 µM. The PT values preferably amount to about 0.5 to 100 µM, especially to about 0.5 to 10 µM. The aPTT values preferably amount to about 0.5 to 100 µM, especially to about 0.5 to 10 µM.

| Example | Ki [µM] factor Xa |
|---|---|
| 1.4 (1S,2R) enantiomer | 0.003 |

The compounds of the present invention and/or their pharmaceutically acceptable salts can be used as medicaments, e.g. in the form of pharmaceutical preparations for enteral, parenteral or topical administration. They can be administered, for example, perorally, e.g. in the form of tablets, coated tablets, dragées, hard and soft gelatine capsules, solutions, emulsions or suspensions, rectally, e.g. in the form of suppositories, parenterally, e.g. in the form of injection solutions or suspensions or infusion solutions, or topically, e.g. in the form of ointments, creams or oils. Oral administration is preferred.

The production of the pharmaceutical preparations can be effected in a manner which will be familiar to any person skilled in the art by bringing the described compounds of the present invention and/or their pharmaceutically acceptable salts, optionally in combination with other therapeutically valuable substances, into a galenical administration form together with suitable, non-toxic, inert, therapeutically compatible solid or liquid carrier materials and, if desired, usual pharmaceutical adjuvants.

Suitable carrier materials are not only inorganic carrier materials, but also organic carrier materials. Thus, for example, lactose, corn starch or derivatives thereof, talc, stearic acid or its salts can be used as carrier materials for tablets, coated tablets, dragées and hard gelatine capsules. Suitable carrier materials for soft gelatine capsules are, for example, vegetable oils, waxes, fats and semi-solid and liquid polyols (depending on the nature of the active ingredient no carriers might, however, be required in the case of soft gelatine capsules). Suitable carrier materials for the production of solutions and syrups are, for example, water, polyols, sucrose, invert sugar and the like. Suitable carrier materials for injection solutions are, for example, water, alcohols, polyols, glycerol and vegetable oils. Suitable carrier materials for suppositories are, for example, natural or hardened oils, waxes, fats and semi-liquid or liquid polyols. Suitable carrier materials for topical preparations are glycerides, semi-synthetic and synthetic glycerides, hydrogenated oils, liquid waxes, liquid paraffins, liquid fatty alcohols, sterols, polyethylene glycols and cellulose derivatives.

Usual stabilizers, preservatives, wetting and emulsifying agents, consistency-improving agents, flavour-improving agents, salts for varying the osmotic pressure, buffer substances, solubilizers, colorants and masking agents and antioxidants come into consideration as pharmaceutical adjuvants.

The dosage of the compounds of the present invention can vary within wide limits depending on the disease to be controlled, the age and the individual condition of the patient and the mode of administration, and will, of course, be fitted to the individual requirements in each particular case. For adult patients a daily dosage of about 1 to 1000 mg, especially about 1 to 300 mg, comes into consideration. Depending on severity of the disease and the precise pharmacokinetic profile the compound could be administered with one or several daily dosage units, e.g. in 1 to 3 dosage units.

The pharmaceutical preparations conveniently contain about 1-500 mg, preferably 1-100 mg, of a compound of the present invention.

The following Examples serve to illustrate the present invention in more detail. They are, however, not intended to limit its scope in any manner.

### Examples

### Example 1

1.1 3-Oxabicyclo[3.1.0]hexane 2-4-dione (1.0 g; CAS 5617-74-3) was dissolved under an argon atmosphere in THF (30 ml). To this solution 1-(4-amino-3-fluorophenyl)-1H-pyridin-2-one (2.0 g; CAS 536747-52-1, prepared according to C. F. Bigge et *al.,* patent application WO 2003045912) were added. The suspension was stirred at r.t. over night, then concentrated. The residue was suspended in 1N HCl. The solid was filtered and washed consecutively with 1N HCl, water and cyclohexane and then dried to give (1RS,2SR)-2-[2-fluoro-4-(2-oxo-2-pyridin-1-yl)-phenylcarbamoyl]-cyclopropanecarboxy-lic acid (1.88 g) as off-white solid. MS 317.1 ([M+H]⁺) 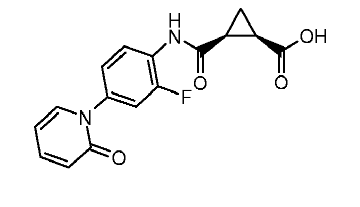
1.2 A suspension of (1RS,2SR)-2-[2-fluoro-4-(2-oxo-2-pyridin-1-yl)-phenylcarbamoyl]-cyclo-propanecarboxylic acid (1.87 g) in MeOH (70 ml) was cooled to 0 °C and then treated with thionylchloride (0.55 ml). The solution was stirred for 3 hrs at 0 °C, then concentrated to give (1RS,2SR)-2-[2-fluoro-4-(2-oxo-2-pyridin-1-yl)-phenylcarbamoyl]-cyclopropanecar-boxylic acid methyl ester (2.1 g) as light yellow solid which was used in the next reaction step without further purification. MS 329.3 ([M-H]⁻) 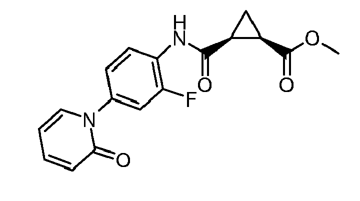
1.3 A solution of 2-amino-5-chloropyridine (3.3. g) in dioxane (30 ml) was treated at r.t. under an argon atmosphere with trimethylaluminium solution (13 ml; 2M in heptane). The reaction mixture was stirred for 2 hrs at r.t.. A solution of (1RS,2SR)-2-[2-fluoro-4-(2-oxo-2-pyridin-1-yl)-phenylcarbamoyl]-cyclopropanecarboxylic acid methyl ester (2.1 g) in dioxane (30 ml) was added. The reaction mixture was heated to 100 °C over night. Then, 12 ml water were added. After stirring for 15 min at r.t., Na₂SO₄ was added. Stirring was continued for another 15 min. Then the solid was filtered off and washed with CH₂Cl₂. The filtrate was concentrated. The crude product was purified by chromatography (silica gel; gradient: CH₂Cl₂ -> CH₂Cl₂/MeOH 95:5) to give (1RS,2SR)-cyclopropane-1,2-dicarboxylic acid 1-[(5-chloro-pyridin-2-yl)-amide] 2-{[2-fluoro-4-(2-oxo-2-pyridin-1-yl)-phenyl]-amide} (2.3 g) as yellow solid. MS 425.0 ([M-H]⁻) 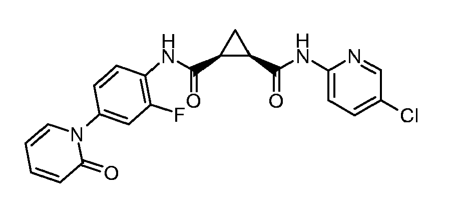
1.4 A solution of (1RS,2SR)-cyclopropane-1,2-dicarboxylic acid 1-[(5-chloropyridin-2-yl)-amide] 2-{[2-fluoro-4-(2-oxo-2-pyridin-1-yl)-phenyl]-amide} (442 mg) in CH₂Cl₂/MeOH 2:1 (minimal amount required to obtain a clear solution) was applied to a HPLC system using a Chiralcel OD stationary phase and 30% isopropanol in heptane as eluent. The first eluting enantiomer was concentrated.

The residue was triturated with tert-butyl methylether, then filtrated and dried to give (1S,2R)-1-methyl-cyclopropane-1,2-dicarboxylic acid 2-[(5-chloro-pyridin-2-yl)-amide] 1-{[2-fluoro-4-(2-oxo-2H-pyridin-1-yl)-phenyl]-amide} (126 mg) as white solid. Enantiomeric purity: 99% ee.

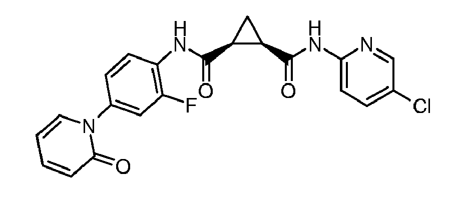

The second eluting enantiomer was isolated by an analogous procedure to give (1R,2S)-1-methyl-cyclopropane-1,2-dicarboxylic acid 2-[(5-chloro-pyridin-2-yl)-amide] 1-{[2-fluoro-4-(2-oxo-2H-pyridin-1-yl)-phenyl]-amide} (118 mg) as white solid. Enantiomeric purity: 78% ee.

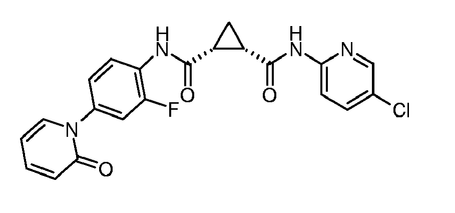

### Example 2

2.1 A solution of 1-methyl-cyclopropane-1,2-dicarboxylic acid dimethyl ester (4.5 g; JACS 1958, 80, 6568) in MeOH (20 ml) and water (20 ml) was treated with 3.1 g NaOH. The reaction mixture was stirred over night at 50 °C, then concentrated. The residual white solid was dissolved in water (25 ml) and washed with diethyl ether (25 ml). The aqueous layer was brought to pH 1 with 3N HCl, then extracted EtOAc. The organic extract was dried (MgSO₄), filtrated and concentrated to give (1SR,2RS)-1-methyl-cyclopropane-1,2-dicarboxylic acid (3.2 g) as white solid which was used in the next reaction step without further purification. 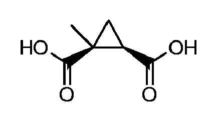
2.2 The starting material (1SR,2RS)-1-methyl-cyclopropane-1,2-dicarboxylic acid (3.2 g) was treated at 0 °C and under an argon atmosphere with trifluoroacetic anhydride. The reaction mixture was stirred at 0 °C for 2 hrs, then concentrated and evaporated three times from THF to give (1SR,5RS)-1-methyl-3-oxabicyclo[3.1.0]hexane-2,4-dione (2.9 g) as light yellow liquid which was used in the next reaction step without further purification. 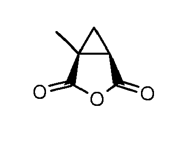
2.3 A solution of (1SR,5RS)-1-methyl-3-oxa-bicyclo[3.1.0]hexane-2,4-dione (2.9 g) in THF (30 ml) was treated at 0 °C and under an argon atmosphere with 2-amino-5-chloropyridine. The reaction mixture (first a solution, then a suspension) was stirred at 0 °C for 1 hr, then at r.t. overnight. The mixture was taken up in water and extracted with EtOAc. The organic layer was dried (MgSO₄), filtrated, concentrated and recrystallized twice from CH₂Cl₂ to give (1SR,2RS)-2-(5-chloropyridin-2-ylcarbamoyl)-1-methyl-cyclopropanecarboxylic acid (2.6 g) as white solid. MS 255.3 ([M-H]⁻) 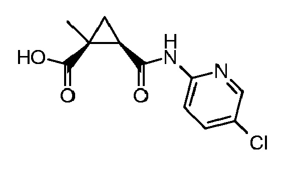
2.4 A solution of (1SR,2RS)-2-(5-chloro-pyridin-2-ylcarbamoyl)-1-methylcyclopropanecarboxylic acid (2.2 g) in MeOH (50 ml) was treated at 0 °C and under an Argon atmosphere with thionyl chloride (1.5 ml). The reaction mixture was stirred at r.t. overnight, then concentrated. The crude product was purified by chromatography (silica gel; gradient: CH₂Cl₂ -> CH₂Cl₂/MeOH 95:5) to give (1SR,2RS)-2-(5-chloro-pyridin-2-ylcarbamoyl)-1-methyl-cyclopropanecarboxylic acid methyl ester (2.1 g) as white solid. MS 269.4 ([M+H]⁺) 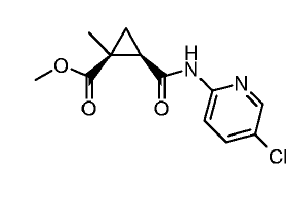
2.5 A solution of 1-(4-amino-3-fiuoro-phenyl)-1H-pyridin-2-one (0.61 g; CAS 536747-52-1, prepared according to C. F. Bigge et *al.,* patent application WO 2003045912) in dioxane (4 ml) was treated at r.t. under an argon atmosphere with trimethylaluminium solution (1.49 ml; 2M in heptane). The reaction mixture was stirred for 2 hrs at r.t.. A solution of (1SR,2RS)-2-(5-chloro-pyridin-2-ylcarbamoyl)-1-methyl-cyclopropanecar-boxylic acid methyl ester (0.2 g) in dioxane (4 ml) was added. The reaction mixture was heated to 100 °C over night, then cooled to r.t. and treated with 0.8 ml H₂O. After stirring for 15 min at r.t., Na₂SO₄ was added. Stirring was continued for another 15 min. Then the solid was filtered off and washed with CH₂Cl₂. The filtrate was washed with 1N HCl. The organic layer was dried (MgSO₄) and concentrated. The crude product was purified by chromatography (silica gel; gradient: CH₂Cl₂ -> CH₂Cl₂/MeOH 95:5) to give (1SR,2RS)-1-methyl-cyclopropane-1,2-dicarboxylic acid 2-[(5-chloro-pyridin-2-yl)-amide] 1-{[2-fluoro-4-(2-oxo-pyridin-1-yl)-phenyl]-amide} (0.175 g) as yellow solid. MS 439.1 ([M+H]⁺) 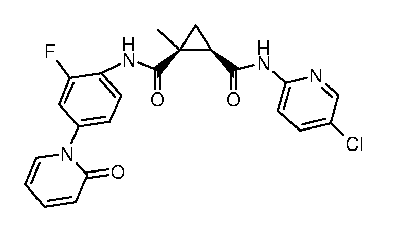
2.6 (1SR,2RS)-1-Methyl-cyclopropane-1,2-dicarboxylic acid 2-[(5-chloropyridin-2-yl)-amide] 1-{[2-fluoro-4-(2-oxo-pyridin-1-yl)-phenyl]-amide}(170 mg) was separated into its enantiomers 1-Methyl-cyclopropane-1,2-dicarboxylic acid 2-[(5-chloro-pyridin-2-yl)-amide] (1S,2R)-1-{[2-fluoro-4-(2-oxo-2H-pyridin-1-yl)-phenyl]-amide} and (1R,2S)-1-methyl-cyclopropane-1,2-dicarboxylic acid 2-[(5-chloro-pyridin-2-yl)-amide] 1-{[2-fluoro-4-(2-oxo-2H-pyridin-1-yl)-phenyl]-amide} by preparative HPLC on a Chiralcel OD stationary phase using 20% EtOH in heptane as eluent.
First eluting enantiomer: 45 mg, off-white solid. MS 439.3 ([M-H]⁻)
Second eluting enantiomer: 76 mg, off-white solid. MS 439.3 ([M-H]⁻)

The configuration of both enantiomers is unassigned.

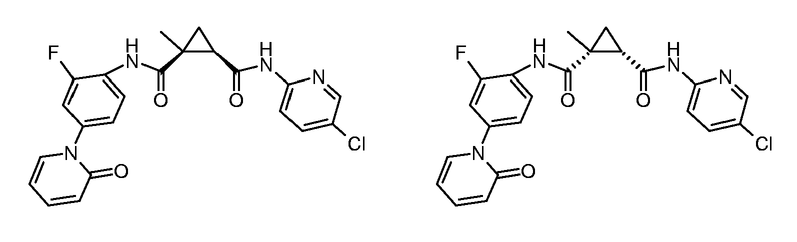

### Example A

Film coated tablets containing the following ingredients can be manufactured in a conventional manner:

| Ingredients | Per tablet | |
|---|---|---|
| Kernel: | | |
| Compound of the present invention | 10.0 mg | 200.0 mg |
| Microcrystalline cellulose | 23.5 mg | 43.5 mg |
| Lactose hydrous | 60.0 mg | 70.0 mg |
| Povidone K30 | 12.5 mg | 15.0 mg |
| Sodium starch glycolate | 12.5 mg | 17.0 mg |
| Magnesium stearate | 1.5 mg | 4.5 mg |
| (Kernel Weight) | 120.0 mg | 350.0 mg |

| Film Coat: | | |
|---|---|---|
| Hydroxypropyl methyl cellulose | 3.5 mg | 7.0 mg |
| Polyethylene glycol 6000 | 0.8 mg | 1.6 mg |
| Talc | 1.3 mg | 2.6 mg |
| Iron oxyde (yellow) | 0.8 mg | 1.6 mg |
| Titan dioxide | 0.8 mg | 1.6 mg |

The active ingredient is sieved and mixed with microcristalline cellulose and the mixture is granulated with a solution of polyvinylpyrrolidon in water. The granulate is mixed with sodium starch glycolate and magesiumstearate and compressed to yield kernels of 120 or 350 mg respectively. The kernels are lacquered with an aqueous solution / suspension of the above mentioned film coat.

### Example B

Capsules containing the following ingredients can be manufactured in a conventional manner:

| Ingredients | Per capsule |
|---|---|
| Compound of the present invention | 25.0 mg |
| Lactose | 150.0 mg |
| Maize starch | 20.0 mg |
| Talc | 5.0 mg |

The components are sieved and mixed and filled into capsules of size 2.

### Example C

Injection solutions can have the following composition:

| | |
|---|---|
| Compound of the present invention | 3.0 mg |
| Polyethylene Glycol 400 | 150.0 mg |
| Acetic Acid | q.s. ad pH 5.0 |
| Water for injection solutions | ad 1.0 ml |

The active ingredient is dissolved in a mixture of Polyethylene Glycol 400 and water for injection (part). The pH is adjusted to 5.0 by Acetic Acid. The volume is adjusted to 1.0 ml by addition of the residual amount of water. The solution is filtered, filled into vials using an appropriate overage and sterilized.

### Example D

### Soft gelatin capsules containing the following ingredients can be manufactured in a conventional manner:

### Capsule contents

| | |
|---|---|
| Compound of the present invention | 5.0 mg |
| Yellow wax | 8.0 mg |
| Hydrogenated Soya bean oil | 8.0 mg |
| Partially hydrogenated plant oils | 34.0 mg |
| Soya bean oil | 110.0 mg |
| Weight of capsule contents | 165.0 mg |

| Gelatin capsule | |
|---|---|
| Gelatin | 75.0 mg |
| Glycerol 85 % | 32.0 mg |
| Karion 83 | 8.0 mg (dry matter) |
| Titan dioxide | 0.4 mg |
| Iron oxide yellow | 1.1 mg |

The active ingredient is dissolved in a warm melting of the other ingredients and the mixture is filled into soft gelatin capsules of appropriate size. The filled soft gelatin capsules are treated according to the usual procedures.

### Example E

Sachets containing the following ingredients can be manufactured in a conventional manner:

| | |
|---|---|
| Compound of the present invention | 50.0 mg |
| Lactose, fine powder | 1015.0 mg |
| Microcristalline cellulose (AVICELPH 102) | 1400.0 mg |
| Sodium carboxymethyl cellulose | 14.0 mg |
| Polyvinylpyrrolidon K 30 | 10.0 mg |
| Magnesiumstearate | 10.0 mg |
| Flavoring additives | 1.0 mg |

The active ingredient is mixed with lactose, microcristalline cellulose and sodium carboxymethyl cellulose and granulated with a mixture of polyvinylpyrrolidon in water. The granulate is mixed with magnesiumstearate and the flavouring additives and filled into sachets.

## Claims

1. Compounds of formula (I) 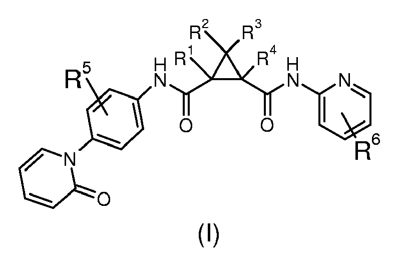 wherein
R² and R³ are independently from each other hydrogen, C₁₋₆ alkyl, carboxyl, C₁₋₆ alkoxycarbonyl, carbamoyl, mono- or di-substituted amino-carbonyl, optionally substituted aryl carbonyl, optionally substituted heterocyclylcarbonyl, optionally substituted heteroarylcarbonyl, optionally substituted aryl, optionally substituted heteroaryl, optionally substituted heterocyclyl, hydroxyl C₁₋₆ alkyl, halo C₁₋₆ alkyl, cyano C₁₋₆ alkyl, C₁₋₆ alkoxy C₁₋₆ alkyl, amino C₁₋₆ alkyl, mono- or di-substituted amino-C₁₋₆ alkyl, optionally substituted aryl C₁₋₆ alkyl, optionally substituted heterocyclyl C₁₋₆ alkyl, optionally substituted heteroaryl C₁₋₆ alkyl, optionally substituted aryl C₁₋₆ alkoxy C₁₋₆ alkyl, optionally substituted heteroaryl C₁₋₆ alkoxy C₁₋₆ alkyl, optionally substituted heterocyclyl C₁₋₆ alkoxy C₁₋₆ alkyl,
R¹ and R⁴ are, independently from each other hydrogen, C₁₋₆ alkyl, cyano, C₁₋₆ alkoxycarbonyl, C₂₋₆ alkenyloxycarbonyl, C₂₋₆ alkynyloxycarbonyl, hydroxyl C₁₋₆ alkyl, C₁₋₆ alkoxycarbonyl, carboxyl, mono- or di-C₁₋₆ alkyl substituted amino-carbonyl, aminocarbonyl, optionally substituted heterocyclyl carbonyl, optionally substituted heteroaryl carbonyl or optionally substituted aryl carbonyl,
R⁵ and R⁶ are, independently from each other, are chlorine, fluorine or bromine;
or pharmaceutically acceptable salts thereof.

2. The compounds according to claim 1, wherein R² and R³ are independently from each other hydrogen, C₁₋₆ alkyl, carboxyl, C₁₋₆ alkoxycarbonyl, carbamoyl, mono- or di-substituted amino-carbonyl, optionally substituted heterocyclylcarbonyl, optionally substituted heteroarylcarbonyl, aryl, optionally substituted heteroaryl, optionally substituted heterocyclyl, hydroxyl C₁₋₆ alkyl, C₁₋₆ alkoxy C₁₋₆ alkyl, amino C₁₋₆ alkyl, mono- or di-substituted amino-C₁₋₆ alkyl, optionally substituted heterocyclyl C₁₋₆ alkyl or optionally substituted heteroaryl C₁₋₆ alkyl,
R¹ and R⁴ are independently from each other hydrogen or C₁₋₆ alkyl.

3. The compounds according to any one of claims 1 and 2, wherein R⁵ is fluorine and R⁶ is chlorine.

4. The compounds according to any one of claims 1 to 3, which is
(1S,2R)-1-methyl-cyclopropane-1,2-dicarboxylic acid 2-[(5-chloro-pyridin-2-yl)-amide] 1-{[2-fluoro-4-(2-oxo-2H-pyridin-1-yl)-phenyl]-amide},
(1R,2S)-1-methyl-cyclopropane-1,2-dicarboxylic acid 2-[(5-chloro-pyridin-2-yl)-amide] 1-{[2-fluoro-4-(2-oxo-2H-pyridin-1-yl)-phenyl]-amide},
1-methyl-cyclopropane-1,2-dicarboxylic acid 2-[(5-chloro-pyridin-2-yl)-amide] (1S,2R)-1-{[2-fluoro-4-(2-oxo-2H-pyridin-1-yl)-phenyl]-amide}, or
(1R,2S)-1-methyl-cyclopropane-1,2-dicarboxylic acid 2-[(5-chloro-pyridin-2-yl)-amide] 1-1[2-fluoro-4-(2-oxo-2H-pyridin-1-yl)-phenyl]-amidel.

5. Pharmaceutical compositions comprising the compound according to any one of claims 1 to 4 and a pharmaceutically acceptable excipient.

6. The compound according to any one of claims 1 to 4 for use as a therapeutic active substance.

7. The compound according to any one of claims 1 to 4 for use as a therapeutic active substance for the treatment and/or prophylaxis of diseases which are associated with the coagulation factor Xa.

8. Use of the compound according to any one of claims 1 to 4 for the preparation of medicaments for the therapeutic and/or prophylactic treatment of diseases which are associated with the coagulation factor Xa.

9. The use according to claim 8, wherein the disease is thrombotic disorders, arterial thrombosis, venous thrombosis, deep vein thrombosis, peripheral arterial occlusive disease, unstable angina pectoris, myocardial infarction, coronary artery disease, pulmonary embolism, stroke due to atrial fibrillation, inflammation, arteriosclerosis, acute vessel closure associated with thrombolytic therapy or restenosis, and/or tumour.
